# EUROPEAN PATENT SPECIFICATION

(11) **EP 3 089 687 B1**
(45) Date of publication and mention of the grant of the patent: **06.06.2018**
(21) Application number: 15704691.3
(22) Date of filing: 10.02.2015
(51) Int. Cl.: A61B 18/14, A61B 18/00, A61B 17/00, A61B 18/12, A61M 25/01, A61N 1/05

(54) **ABLATION CATHETER**
ABLATIONSKATHETER
CATHÉTER D'ABLATION

(30) Priority: 11.02.2014 US 201461938417 P
(43) Date of publication of application: 09.11.2016
(73) Proprietor: St. Jude Medical, Cardiology Division, Inc., St. Paul MN 55117 (US)
(72) Inventor: WITTKAMPF, Frederik H.M., 3749AJ Lage Vuursche (NL); MONAHAN, Brian, Elk River, MN 55330 (US)
(74) Representative: Kramer Barske Schmidtchen Patentanwälte PartG mbB
(86) International application number: PCT/US2015/015116
(87) International publication number: WO 2015/123163

(56) References cited:
- EP-A1- 2 241 279
- WO-A1-2005/025438
- WO-A2-2007/005641
- CA-A1- 2 586 022
- US-A- 5 454 370
- US-A1- 2005 261 672

## Description

### FIELD

The present disclosure relates to medical catheters for electrically isolating tissue, and more particularly to catheters for delivering ablation energy via multiple electrodes arranged in a plane substantially aligned with or otherwise parallel to a longitudinal axis of the catheter.

EP 2 241 279 A1 discloses an epicardial mapping and ablation catheter. US 2005/0261672 A1 discloses systems and methods for selective denervation of heart dysrhythmias. US 5,454,370 A discloses a mapping and ablation electrode configuration.

### SUMMARY

According to the invention, a catheter according to claim 1 and a system according to claim 7 are provided. Preferred embodiments are defined in the dependent claims. A method involving positioning a plurality of electrodes on a distal portion of an ablation catheter shaft is also disclosed herein, however, this method does not form part of the invention.

### BRIEF DESCRIPTION OF THE DRAWINGS

FIGs. 1A and 1B depict a medical device showing both a shaft and corresponding shaft extension that includes electrodes;
FIGs. 2A and 2B depict another representative embodiment of a medical catheter having a shaft and a shaft extension in the form of a circular extension of the shaft;
FIG. 3 depicts a flexible shaft extension capable of being deformed and of being returned to its pre-deformed shape;
FIGs. 4A-4I depict other representative planar shapes in which the principles described herein may be applied;
FIGs. 5A-5C depict various examples in which the catheters described herein may be deflected;
FIG. 6 illustrates one representative technique for enabling uni-directional or bidirectional (or greater) deflections from the longitudinal axis of the shaft;
FIGs. 7A and 7B depict one embodiment of how the catheters described herein may be implemented in an epicardial therapeutic capacity;
FIG. 8 is a representative example of a system including a generator, a cable, and a catheter having a shaft and a shaft extension with a plurality of electrodes arranged beyond the end of the shaft and in a plane substantially aligned with the shaft axis; and
FIGs. 9A and 9B are flow diagrams of representative methods for creating an ablation catheter in accordance with the disclosure.

### DETAILED DESCRIPTION

In the following description, reference is made to the accompanying drawings that depict representative examples. It is to be understood that other embodiments and implementations may be utilized, as structural and/or operational changes may be made without departing from the scope of the disclosure. Like reference numbers are used throughout the disclosure where appropriate.

The disclosure is generally directed to medical devices. Devices and techniques are disclosed that enable multiple electrodes to be positioned proximate organic tissue, such as human tissue. The electrodes may be used to, for example, pass energy to ablate the tissue. In one embodiment, the ablation is performed using direct current (DC) or alternating current (AC) current, such that an appropriate quantity of energy can irreversibly electroporate cells of the tissue, which can address physiological issues such as, for example, atrial fibrillation or flutter, ventricular tachycardia, and/or other electrophysiological issues in addition to other issues treatable by ablation (e.g. renal denervation, etc.). More particularly, an externally applied electric field is applied to a cell which causes the cell wall to become permeable. If the pulse duration and wave form exceed the voltage threshold for the cell membrane, the cell wall is irreversibly damaged this process is known as irreversible electroporation (IRE). While embodiments described herein may be described in terms of cardiac treatments, the disclosure is not limited thereto.

For example, in one embodiment a medical catheter is provided that includes a shaft and a distal segment. The distal segment of the shaft includes a plurality of electrodes that are configured in a plane arranged to deviate from the longitudinal axis of the shaft, where the electrode plane is substantially aligned with the longitudinal axis of the shaft. This arrangement provides, among other things, one manner of positioning the catheter electrodes against tissue in situations where the catheter can be moved along the tissue surface. One representative example of such a situation is in connection with epicardial ablation procedures, where the pericardium is intentionally breached in order to advance the medical catheters described herein to the epicardial surface and position the electrodes against the tissue for electroporation ablation procedures.

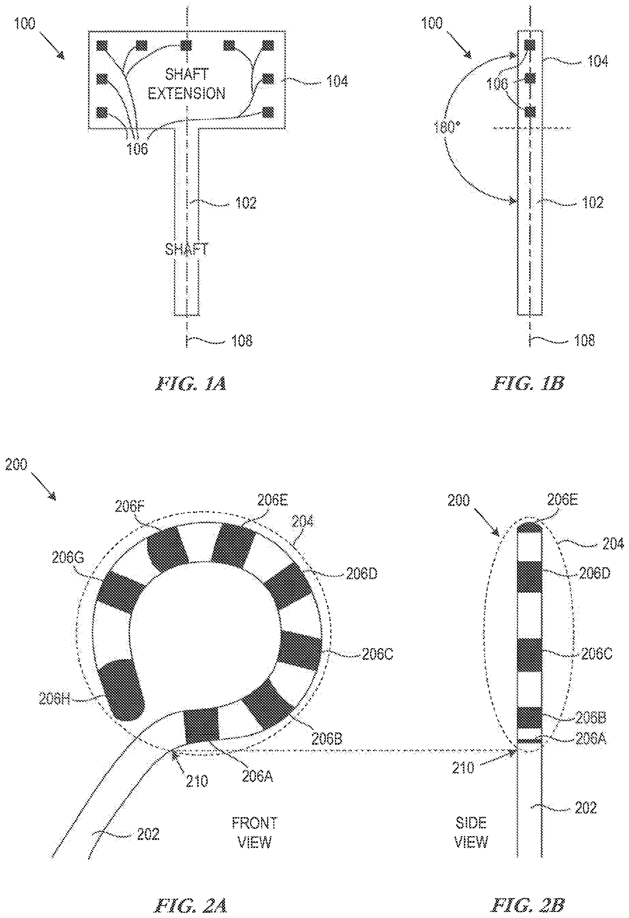

FIG. 1A is a block diagram of one embodiment of a medical device 100 in accordance with the disclosure. In this embodiment, a front view of the medical device 100 depicts at least a shaft 102 and a shaft extension 104. The shaft may represent a catheter shaft, such as a flexible epicardial catheter shaft capable of being introduced (by way of separate introducer or not) into a body such that the shaft extension 104 is positioned proximate a tissue target, such as the epicardium in order to perform epicardial ablation procedures.

In the embodiment of FIG. 1A, the shaft extension includes a plurality of electrodes 106. In one embodiment, each of the electrodes 106 is coupled to a respective conductor (not shown) such as a respective current-carrying wire. By applying energy to the electrodes 106 by way of the conductors, tissue necrosis on which the electrodes 106 are positioned can be effected. For example, in a radio frequency (RF) embodiment, RF energy can be passed to the electrodes 106, which enables tissue to be heated such that tissue necrosis can impact undesirable electrical impulses that trigger abnormal cardiac activity. Other types of ablation may also be effected, such as cryoablation, DC ablation, etc.

In one embodiment, the catheters described herein facilitate DC or AC ablation techniques, such as causing tissue necrosis by way of irreversible electroporation through application of current to the tissue. By applying a sufficiently high electrical shock to the catheter electrodes, the tissue areas contacting the tissue delivery locations become permanently nonconductive. Furthermore, by using a plurality of shock delivery locations in close contact with the tissue to be treated, the need for repositioning the catheter multiple times for creating an electrical isolation between two areas of cardiac tissue is reduced. With the devices described herein, a relative long length of cardiac tissue can be treated in a single operation, reducing the procedure time. Such treatments may be applied, for example, during approximately 5 ms of between 200 and 500 Joule.

Positioning a plurality of electrodes proximate tissue to carry out such ablation techniques may be challenging. In accordance with one embodiment, the electrodes 106 of the shaft extension 104 are positioned in a plane, that is, substantially positioned in two dimensions. This plane of electrodes is aligned with the longitudinal axis of the shaft 102. FIG. 1B depicts the medical device 100, showing both the shaft 102 and shaft extension 104 from a side view. As can be seen, the shaft extension 104, which is positioned in a planar fashion, aligns with the shaft 102 such that the two segments 102, 104 are aligned, or parallel. Thus, no significant acute or obtuse angle is formed between the plane of the electrodes 106 of the shaft extension 104 and the longitudinal axis 108 of the shaft 102, and therefore form a 180 degree angle. As depicted in the representative medical catheter 100 of FIGs. 1A and 1B, the catheter 100 includes a shaft 102 and a distal segment represented by the shaft extension 104. where the distal segment includes a plurality of electrodes 106 that are configured in a plane and arranged to deviate from the longitudinal axis of the shaft 108 (see FIG. 1A, where the electrodes 106 are not aligned with the axis 108 in the front view), however where the electrode plane formed by the electrodes 106 is substantially aligned with the longitudinal axis 108 of the shaft (see FIG. 1B).

FIG. 2A is another representative embodiment of a medical catheter 200 having a shaft 202 and a shaft extension 204. In this embodiment, the shaft extension 204 is implemented by a distal portion of the shaft that primarily forms a circular (including oval) shape. According to the invention, this shape is created using memory wire, such as nitinol wire or other shape memory alloy. In this representative embodiment, there are eight electrodes 206A-H on the shaft extension 204, each of which may carry current to an ablation target site to effect the ablation procedures. As seen in the front view of FIG. 2A and corresponding side view of FIG. 2B, this embodiment also involves positioning the electrodes 206A-H in a planar fashion such that the plane of electrodes is substantially aligned with the longitudinal axis of the shaft 202. Thus, the plane of electrodes does not form a significant angle with the shaft 202, thereby enabling the electrode plane to avoid jutting out in therapy situations where this would be undesirable. Another representative embodiment includes an octopolar, 12 mm circular catheter 2with 2 mm ring electrodes. In yet another embodiment, the tip electrode 206H is replaced by a ring electrode, such that all electrodes are ring electrodes.

The radius of the "loop" can be any desired radius. Representative examples include, for example, 15 mm, 18 mm, 20 mm, etc. In other embodiments, an actuator may be provided and structure to vary the loop size, such that manipulation of an actuator expands or reduces the loop radius, such as between 15 mm and 20 mm. In one example embodiment, electrode rings may be, for example, 2 mm, 4 mm, etc.

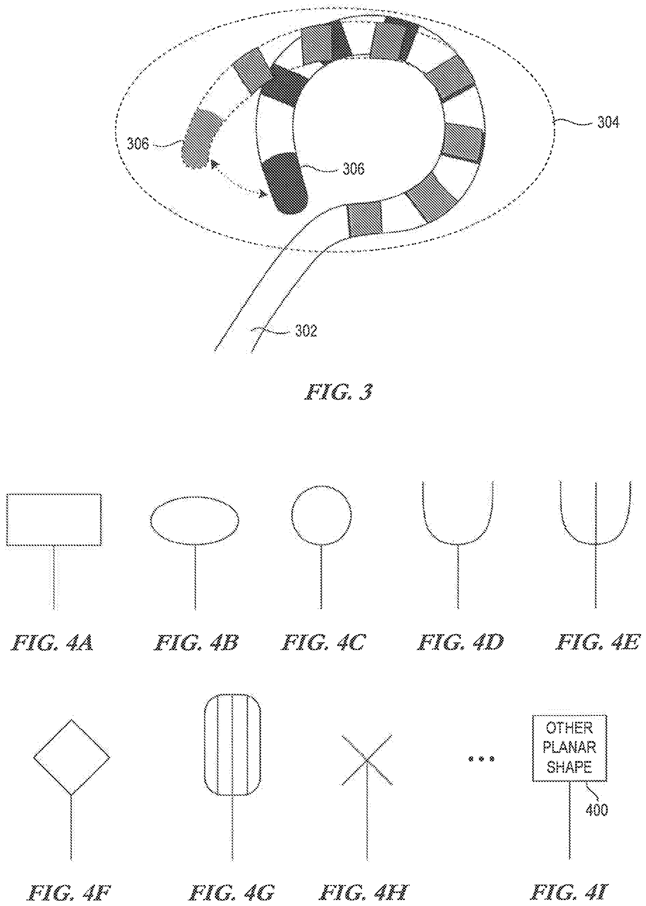

It should be noted that the shaft extension 204 may be flexible. FIG. 3 depicts a flexible shaft extension 304, such that it may be deformed. For example, the shape may be flexible, whereby a force may be experienced by the shaft extension, and after removal of the force causing the shape to flex, it returns to the shape determined by the memory wire. In other embodiments, the shaft extension 304 may be firm and less deformable or not deformable without applying a force that could permanently deform the shaft extension 304.

The shaft extension that houses the plurality of electrodes may be any desired shape that can be formed on a plane. FIGs. 4A-4I depict other representative planar shapes in which the principles described herein may be applied. It should be noted that the examples of FIGs. 4A-4I are presented for purposes of example only, and do not represent an exhaustive list of planar shapes, as indicated by FIG. 4I where any other planar shape 400 may be utilized.

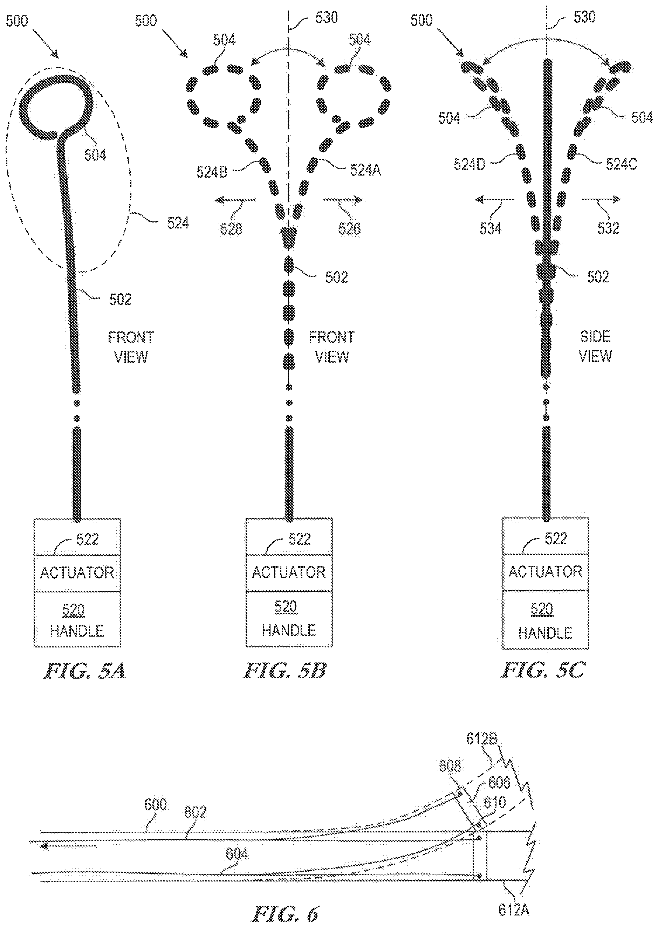

In some embodiments, the catheters described herein may be deflectable. For example, FIGs. 5A, 5B and 5C depict how catheters described herein may be deflected in one or more directions. FIG. 5A depicts a catheter 500 having a shaft 502 and shaft extension in the form of a circular loop 504 having ablation electrodes positioned thereon (not shown). The catheter may be connected to a handle 520 that includes any type of actuator 522 capable of deflecting some distal portion 524 of the catheter that at least includes the shaft extension (circular loop 504 in the example of FIGs. 5A-5C). For example, the actuator 522 may be a rocker arm, plunger, rotating knob, or other mechanism coupled to one or more deflection wires or "pull wires" (not shown) that are capable of deflecting the distal portion 524.

FIG. 5B depicts an embodiment where the distal portion 524 is capable of deflection in one or two directions substantially in the plane of the circular loop 504. Thus, from a front view of the catheter 500, the distal portion could be deflected from a longitudinal axis 530 in a first direction 526 and/or second direction 528 as depicted by deflected distal portions 524A, 524B respectively. FIG. 5C depicts another embodiment where the distal portion 524 is capable of deflection in one or two directions substantially perpendicular to the plane of the circular loop 504. Thus, from a side view of the catheter 500, the distal portion could be deflected form the longitudinal axis 530 in a first direction 532 and/or second direction 534 as depicted by deflected distal portions 524C, 524D respectively. It should be recognized that deflection can be effected in any one, more or all of directions 526, 528, 532, 534 and/or still additional directions. Known techniques for deflecting catheters may be utilized.

FIG. 6 depicts one embodiment in which catheters described herein may be deflected. The representative catheter 600 embodiment of FIG. 6 includes two tethers depicted as pull wires 602, 604 are coupled to a pull ring 606 at points 608, 610. A distal portion of the catheter 600 is deflected when one of the pull wires is tensioned, such as depicted in FIG. 6 where pull wire 602 is tensioned to cause the neutral positioned of distal portion 612A to be deflected to a new position of distal portion 612B. This merely represents one example of how the catheters described herein may be deflected.

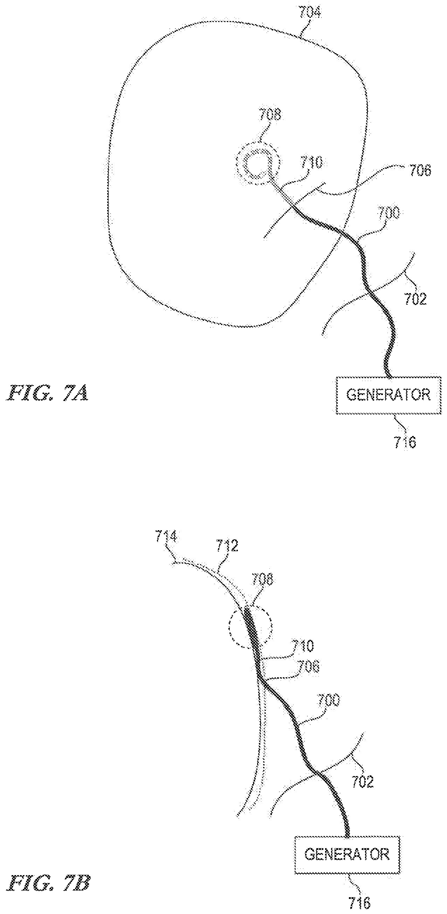

FIG. 7A depicts one embodiment of how the catheters described herein may be implemented. In this example, the catheter 700 is used to perform epicardial ablation. Line 702 represents the entry point of a human body. When reaching the heart 704, an entry point 706 is created in the pericardium by slitting the pericardium to enable the electrode-equipped distal portion 708 of the catheter 700 to be positioned against the epicardial surface. Since the distal portion 708 of the catheter is configured in a plane that is parallel to a longitudinal axis of the catheter 700 (at least the portion of the catheter 710 near the distal portion 708), the planar distal portion 708 may be moved along the epicardial surface to a target ablation site by moving under the pericardium. This is better depicted in FIG. 7B, where the distal portion 708 is shown below the pericardium 712 and the epicardial surface 714. When positioned in this manner at the desired target site, the electrodes (not shown) at the distal portion 708 may be energized by, for example, the generator 716 to pass current through the electrodes and into the proximate tissue.

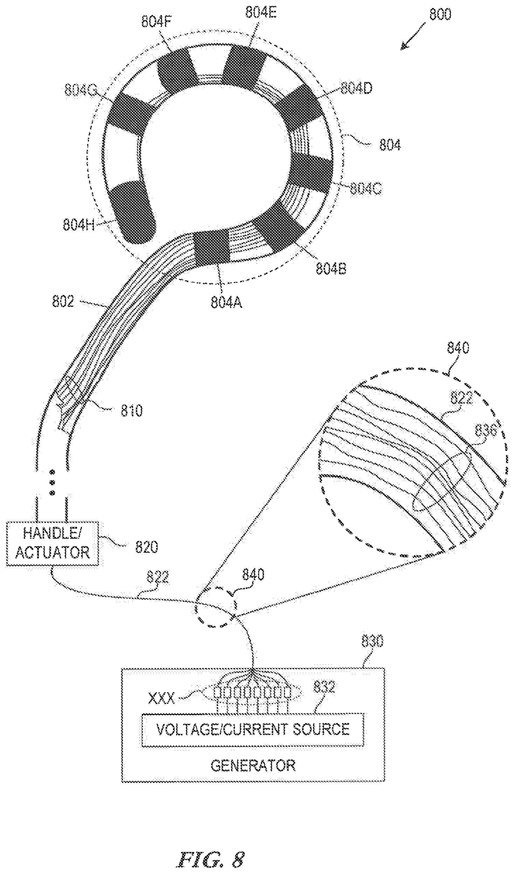

FIG. 8 is a representative example of a system including a catheter 800 having a shaft 802, and a shaft extension 804 with a plurality of electrodes 806A-H arranged beyond the end of the shaft 802 and in a plane substantially aligned with the shaft 802 axis. The system further includes a handle 820 and a generator 830. In one embodiment, the generator 830 represents a DC and/or AC voltage generator 832 that can generate one or more pulses of energy or "shocks." In one embodiment, the voltage generator 832 can perform analogously to a defibrillator, where a monophasic or biphasic pulse or series of pulses of energy can be delivered.

As depicted in the example of FIG. 8, the voltage generator 832 provides energy to each of the conductors 810 that respectively connect to the electrodes 806A-H. A cable(s) 822 can be coupled between a connector 834 of the generator 830 and the handle 820. A breakout view 840 of a portion of such a cable 822 is depicted, where the cable 822 includes conductors 835 from the generator that are respectively coupled to the conductors 812 at the handle/actuator 820 (connections not shown). For example, the handle 820 may include a connector capable of receiving the conductors 836, and capable of receiving the conductors 810, where the conductors 836 are connected one-to-one to conductors 810, thereby providing energy from the generator 830 to each of the electrodes 806A-H.

In one embodiment, current is sourced from the generator 830, and passed from one or more of the electrodes 804A-H, and returned via a return path. The return path may be provided via a body patch, another catheter in the area, an electrode on an introducer/sheath, etc.

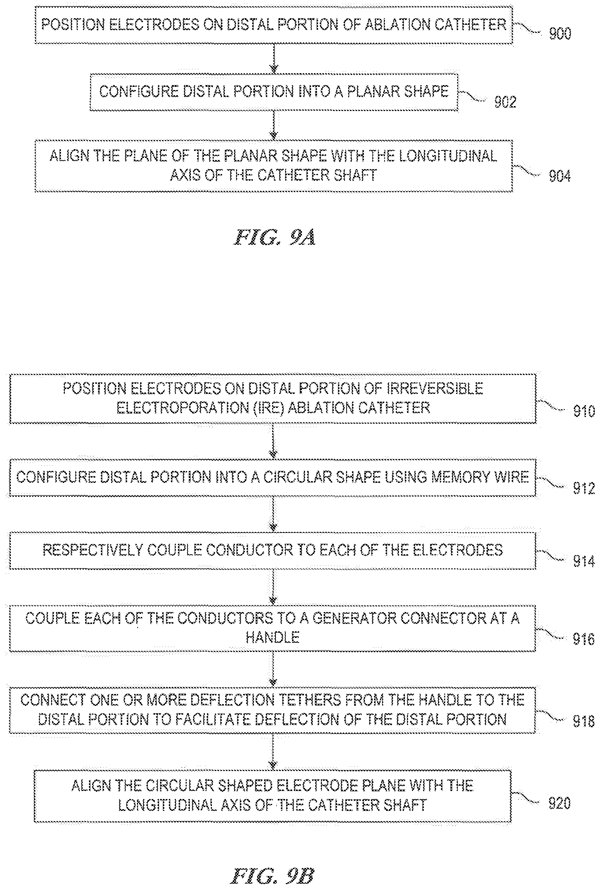

FIG. 9A is a flow diagram of one representative manner for creating an ablation catheter. In the illustrated embodiment, electrodes are positioned 900 on a distal portion of an ablation catheter. The distal portion is configured 902 into a planar shape, and the plane of the planar shape is aligned 904 with the longitudinal axis of the catheter shaft. In this manner, the plurality of electrodes does not create an angle relative to shaft, to facilitate particular uses of the catheter.

FIG. 9B is a flow diagram of another representative manner for creating an ablation catheter. In the illustrated embodiment, the electrodes are positioned 910 on a distal portion of an irreversible electroporation (IRE) ablation catheter. The distal portion is configured 912 into a circular shape using, for example, memory wire such as nitinol. A conductor is respectively coupled 914 to each of the electrodes, and each of the conductors is coupled 916 to a generator connector(s) at a catheter handle. One or more deflection tethers (e.g. wires) are connected 918 from the handle to the distal portion to facilitate deflection of the distal portion. The plane of the circular-shaped electrode plane is aligned 920 with the longitudinal axis of the catheter shaft. In this manner, the plurality of electrodes does not create an angle relative to shaft, to facilitate particular uses of the catheter.

Although the subject matter has been described in language specific to structural features and/or methodological acts, it is to be understood that the subject matter defined in the appended claims is not necessarily limited to the specific features or acts described above. Rather, the specific features and acts described above are disclosed as representative forms of implementing the claims.

## Claims

1. A catheter comprising:
a shaft (102; 202; 302; 502; 802); and
a distal segment (104; 204; 304; 504; 804) of the shaft having a plurality of electrodes (106; 206A-H; 306; 806A-H), wherein the distal segment and the plurality of electrodes are configured in a plane that is substantially parallel with the longitudinal axis of the shaft, and
wherein the plurality of electrodes are configured to accommodate direct current (DC) currents capable of irreversibly electroporating cells in organic tissue, **characterised in that** the distal segment of the shaft deviates from a straight path and is configured using memory wire into a planar shape, and **in that** the shape is flexible, and after removal of a force causing the shape to flex, returns to the planar shape determined by the memory wire.

2. The catheter of claim 1, further comprising a plurality of conductors (810), one for each of the plurality of electrodes, through which current may be passed to energize the respective ones of the plurality of electrodes.

3. The catheter of claim 1 or 2, wherein the distal segment (204; 304) of the shaft (202; 302) is configured in a circular shape.

4. The catheter of claim 1, further comprising:
a deflectable segment (524; 612A) of the shaft that includes the distal segment (504; 804) of the shaft;
a first manipulatable deflection tether coupled to the deflectable segment; and
wherein increasing tension on the first manipulatable deflection tether causes the deflectable segment of the shaft (502; 802) to deflect in a first direction.

5. The catheter of claim 4, further comprising a second manipulatable deflection tether coupled to the deflectable segment opposite the coupling of the first manipulatable deflection tether, wherein increasing tension on the first manipulatable deflection tether causes the deflectable segment of the shaft (802) to deflect in a second direction.

6. The catheter of claim 5, further comprising an nth manipulatable deflection tether coupled to the deflectable segment between other manipulatable deflection tethers, wherein increasing tension on the nth manipulatable deflection tether causes the deflectable segment of the shaft (802) to deflect in an nth direction.

7. A system comprising
a catheter (800) of any one of claims 1 to 6,
a direct current (DC) voltage source (832), and
at least one cable (822) coupled between the DC voltage source and the plurality of electrodes on the catheter (800).

8. The system of claim 7, further comprising a medical introducer configured to pass the catheter (800) therethrough to an ablation target.

## Patentansprüche

1. Katheter, mit
einem Schaft (102; 202; 302; 502; 802), und
einem distalen Segment (104; 204; 304; 504; 804) des Schafts, das eine Mehrzahl von Elektroden (106; 206A-H; 306A-H) aufweist, bei dem das distale Segment und die Mehrzahl an Elektroden in einer Ebene konfiguriert sind, die im Wesentlichen parallel zu der Längsachse des Schaftes ist, und
bei dem die Mehrzahl an Elektroden derart konfiguriert sind, dass sie Gleichstrom (DC)-Ströme aufnehmen, die Zellen in einem organischen Gewebe irreversibel elektroporieren können,
**dadurch gekennzeichnet, dass**
das distale Segment des Schaftes von einem geraden Weg abweicht und unter Verwendung eines Formgedächtnisdrahts in einer planaren Form konfiguriert ist, und
dass die Form flexibel ist und nach Aufheben einer Kraft, die bewirkt, dass sich die Form verbiegt, in die planare Form zurückkehrt, die durch den Formgedächtnisdraht bestimmt wird.

2. Katheter nach Anspruch 1, der ferner eine Mehrzahl an Leitern (810) aufweist, einer für jede der Mehrzahl von Elektroden, durch welchen Strom zur Energieversorgung der jeweiligen einen der Mehrzahl von Elektroden fließt.

3. Katheter nach Anspruch 1 oder 2, bei dem das distale Segment (204; 304) des Schafts (202; 302) in einer Kreisform konfiguriert ist.

4. Katheter nach Anspruch 1, der ferner
ein auslenkbares Segment (524; 612A) des Schaftes, das das distale Segment (504; 804) des Schaftes aufweist,
einen ersten manipulierbaren Auslenkungsdraht aufweist, der mit dem auslenkbaren Segment verbunden ist, und
bei dem ein Erhöhen der Zugspannung an dem ersten manipulierbaren Auslenkungsdraht bewirkt, dass sich das auslenkbare Segment des Schaftes (502; 802) in einer ersten Richtung auslenkt.

5. Katheter nach Anspruch 4, das ferner einen zweiten manipulierbaren Auslenkungsdraht aufweist, der mit dem auslenkbaren Segment gegenüberliegend zu der Koppelung des ersten manipulierbaren Auslenkungsdrahtes gekoppelt ist, bei dem ein Erhöhen der Zugspannung an dem ersten manipulierbaren Auslenkungsdraht bewirkt, dass das auslenkbare Segment des Schaftes (802) in einer zweiten Richtung auslenkt.

6. Katheter nach Anspruch 5, das ferner einen n-ten manipulierbaren Auslenkungsdraht aufweist, der mit dem auslenkbaren Segment zwischen anderen manipulierbaren Auslenkungsdrähten gekoppelt ist, bei dem ein Erhöhen der Zugspannung an dem n-ten manipulierbaren Auslenkungsdraht bewirkt, dass das auslenkbare Segment des Schaftes (802) in einer n-ten Richtung auslenkt.

7. System, mit
einem Katheter (800) nach einem der Ansprüche 1 bis 6,
einer Gleichstrom-(DC)-Spannungsquelle (832), und
zumindest einem Kabel (822), das zwischen der Gleichstromspannungsquelle und der Mehrzahl an Elektroden an den Katheter (800) gekoppelt ist.

8. System nach Anspruch 7, das ferner eine medizinische Einführvorrichtung aufweist, die dazu konfiguriert ist, durch den Katheter (800) zu einem Ablationsziel zu passieren.

## Revendications

1. Cathéter comprenant :
une tige (102 ; 202 ; 302 ; 502 ; 802) ; et
un segment distal (104 ; 204 ; 304 ; 504 ; 804) de la tige ayant une pluralité d'électrodes (106 ; 206A à H ; 306 ; 806A à H), dans lequel le segment distal et la pluralité d'électrodes sont configurés dans un plan qui est sensiblement parallèle à l'axe longitudinal de la tige, et
dans lequel la pluralité d'électrodes est configurée pour s'adapter à des courants continus (CC) aptes à une électroporation irréversible de cellules dans un tissu organique,
**caractérisé en ce que**
le segment distal de la tige s'écarte d'un chemin droit et est configuré à l'aide d'un fil à mémoire selon une forme plane, et
**en ce que** la forme est flexible, et après élimination d'une force amenant la forme à fléchir, retourne à la forme plane déterminée par le fil à mémoire.

2. Cathéter selon la revendication 1, comprenant en outre une pluralité de conducteurs (810), un pour chacune de la pluralité d'électrodes, à travers lesquels le courant peut passer pour alimenter en énergie les électrodes respectives de la pluralité d'électrodes.

3. Cathéter selon la revendication 1 ou 2, dans lequel le segment distal (204 ; 304) de la tige (202 ; 302) est configuré selon une forme circulaire.

4. Cathéter selon la revendication 1, comprenant en outre :
un segment pouvant être défléchi (524 ; 612A) de la tige qui comporte le segment distal (504 ; 804) de la tige ;
une première attache de déflexion manipulable couplée au segment pouvant être défléchi ; et
dans lequel l'augmentation de la tension sur la première attache de déflexion manipulable amène le segment pouvant être défléchi de la tige (502 ; 802) à se défléchir dans une première direction.

5. Cathéter selon la revendication 4, comprenant en outre une deuxième attache de déflexion manipulable couplée au segment pouvant être défléchi à l'opposé du couplage de la première attache de déflexion manipulable, dans lequel l'augmentation de la tension sur la première attache de déflexion manipulable amène le segment pouvant être défléchi de la tige (802) à se défléchir dans une deuxième direction.

6. Cathéter selon la revendication 5, comprenant en outre une nième attache de déflexion manipulable couplée au segment pouvant être défléchi entre d'autres attaches de déflexion manipulables, dans lequel l'augmentation de la tension sur la nième attache de déflexion manipulable amène le segment pouvant être défléchi de la tige (802) à se défléchir dans une nième direction.

7. Système comprenant :
un cathéter (800) de l'une quelconque des revendications 1 à 6,
une source de tension (832) continue (CC), et
au moins un câble (822) couplé entre la source de tension continue et la pluralité d'électrodes sur le cathéter (800).

8. Système selon la revendication 7, comprenant en outre un introducteur médical configuré pour faire passer le cathéter (800) au travers une cible d'ablation.
